# EUROPEAN PATENT APPLICATION

(11) **EP 3 865 584 A1**
(43) Date of publication of application: **18.08.2021**
(21) Application number: 19871204.4
(22) Date of filing: 26.08.2019
(51) Int. Cl.: C12Q 1/68, C40B 50/06

(54) **METHOD FOR CONSTRUCTING SEQUENCING LIBRARY**

(30) Priority: 11.10.2018 CN 201811185409
(71) Applicant: Beijing Euler Technology Limited Company, Beijing 102206 (CN)
(72) Inventor: ZHANG, Yi, Beijing 102206 (CN); CHEN, Qiong, Beijing 102206 (CN)
(74) Representative: Grünecker Patent- und Rechtsanwälte PartG mbB
(86) International application number: PCT/CN2019/102651
(87) International publication number: WO 2020/073748

(57) **Abstract**

Disclosed are a method and kit for constructing a second generation high-throughput sequencing library, wherein same can improve the utilization efficiency of the nucleic acid template and simplify the construction process of the sequencing library to make the sequencing results more accurate and the coverage more uniform.

SEQUENCE LISTING

<110> BEIJING EULER GENOMICS LIMITED COMPANY

<120> METHOD FOR CONSTRUCTING SEQUENCING LIBRARY

<130> EP134030-FZ200

<140> EP19871204.4
<141> 2019-08-26

<150> PCT/CN2019/102651
<151> 2019-08-26
<150> CN201811185409
<151> 2018-10-11

<160> 29

<170> BiSSAP 1.3.6

<210> 1
<211> 33
<212> DNA
<213> synthetic sequence


<400> 1
agacgtgtgc tcttccgatc tttttttttt ttt                                    33


<210> 2
<211> 34
<212> DNA
<213> synthetic sequence


<400> 2
agatcggaag agcacacgtc tgaactccag tcac                                   34


<210> 3
<211> 34
<212> DNA
<213> synthetic sequence


<400> 3
gtgactggag ttcagacgtg tgctcttccg atct                                   34


<210> 4
<211> 21
<212> DNA
<213> synthetic sequence


<400> 4
gtgactggag ttcagacgtg t                                                 21


<210> 5
<211> 21
<212> DNA

<213> synthetic sequence

<400> 5
acactctttc cctacacgac g                                                21

<210> 6
<211> 42
<212> DNA
<213> synthetic sequence

<220>
<221> misc_feature
<222> 1..8
<223> /note="n is a, c, g, or t"

<400> 6
nnnnnnnnag atcggaagag cacacgtctg aactccagtc ac                         42

<210> 7
<211> 35
<212> DNA
<213> synthetic sequence

<220>
<221> misc_feature
<222> 34..35
<223> /note="n is a, c, g, or t"

<400> 7
acactctttc cctacacgac gctcttccga tctnn                                 35

<210> 8
<211> 36
<212> DNA
<213> synthetic sequence

<220>
<221> misc_feature
<222> 34..36
<223> /note="n is a, c, g, or t"

<400> 8
acactctttc cctacacgac gctcttccga tctnnn                                36

<210> 9
<211> 37
<212> DNA
<213> synthetic sequence

<220>
<221> misc_feature
<222> 34..37
<223> /note="n is a, c, g, or t"

<400> 9
acactctttc cctacacgac gctcttccga tctnnnn                                37

<210> 10
<211> 38
<212> DNA
<213> synthetic sequence

<220>
<221> misc_feature
<222> 34..38
<223> /note="n is a, c, g, or t"

<400> 10
acactctttc cctacacgac gctcttccga tctnnnnn                               38

<210> 11
<211> 39
<212> DNA
<213> synthetic sequence

<220>
<221> misc_feature
<222> 34..39
<223> /note="n is a, c, g, or t"

<400> 11
acactctttc cctacacgac gctcttccga tctnnnnnn                              39

<210> 12
<211> 40
<212> DNA
<213> synthetic sequence

<220>
<221> misc_feature
<222> 34..40
<223> /note="n is a, c, g, or t"

<400> 12
acactctttc cctacacgac gctcttccga tctnnnnnnn                             40

<210> 13
<211> 41
<212> DNA
<213> synthetic sequence

<220>
<221> misc_feature
<222> 34..41
<223> /note="n is a, c, g, or t"

<400> 13
acactctttc cctacacgac gctcttccga tctnnnnnnn n                           41

<210> 14
<211> 42
<212> DNA
<213> synthetic sequence

<220>
<221> misc_feature
<222> 33..42
<223> /note="n is a, c, g, or t"

<400> 14
acactctttc cctacacgac gctcttccga tctnnnnnnn nn                42

<210> 15
<211> 33
<212> DNA
<213> synthetic sequence

<400> 15
agatcggaag agcgtcgtgt agggaaagag tgt                33

<210> 16
<211> 33
<212> DNA
<213> synthetic sequence

<400> 16
acactctttc cctacacgac gctcttccga tct                33

<210> 17
<211> 33
<212> DNA
<213> synthetic sequence

<400> 17
gatcggaaga gcacacgtct gaactccagt cac                33

<210> 18
<211> 58
<212> DNA
<213> synthetic sequence

<400> 18
aatgatacgg cgaccaccga gatctacaca cagtgctaca ctctttccct acacgacg                58

<210> 19
<211> 58
<212> DNA
<213> synthetic sequence

<400> 19

aatgatacgg cgaccaccga gatctacacc gcatgtaaca ctctttccct acacgacg 58

<210> 20
<211> 58
<212> DNA
<213> synthetic sequence

<400> 20
aatgatacgg cgaccaccga gatctacact atcgcagaca ctctttccct acacgacg 58

<210> 21
<211> 58
<212> DNA
<213> synthetic sequence

<400> 21
aatgatacgg cgaccaccga gatctacacg tgcatacaca ctctttccct acacgacg 58

<210> 22
<211> 58
<212> DNA
<213> synthetic sequence

<400> 22
aatgatacgg cgaccaccga gatctacaca gctagtcaca ctctttccct acacgacg 58

<210> 23
<211> 58
<212> DNA
<213> synthetic sequence

<400> 23
aatgatacgg cgaccaccga gatctacacc tagctgaaca ctctttccct acacgacg 58

<210> 24
<211> 50
<212> DNA
<213> synthetic sequence

<400> 24
caagcagaag acggcatacg agatgtctta gggtgactgg agttcagacg 50

<210> 25
<211> 50
<212> DNA
<213> synthetic sequence

<400> 25
caagcagaag acggcatacg agattagctg cagtgactgg agttcagacg 50

<210> 26
<211> 45
<212> DNA
<213> synthetic sequence

<400> 26
aatgatacgg cgaccaccga gatctacact ctttccctac acgac 45

<210> 27
<211> 48
<212> DNA
<213> synthetic sequence

<400> 27
caagcagaag acggcatacg agatttgact gtgactggag ttcagacg 48

<210> 28
<211> 48
<212> DNA
<213> synthetic sequence

<400> 28
caagcagaag acggcatacg agattgacat gtgactggag ttcagacg 48

<210> 29
<211> 41
<212> DNA
<213> synthetic sequence

<220>
<221> misc_feature
<222> 21..28
<223> /note="n is a, c, g, or t"

<400> 29
agacgtgtgc tcttccgatc nnnnnnnntt tttttttttt t 41

## Description

### Technical Field

The present invention relates to a method and kit for constructing a second-generation high-throughput sequencing library. More particularly, the present invention relates to a method and kit for constructing a high-throughput sequencing library with a sequencing adapter with random bases overhanging at a 3' terminus.

### Background Art

Compared with first-generation sequencing technology, second-generation sequencing technology has a faster sequencing speed and higher throughput, which meets the requirements of current scientific and technological development with regard to sequencing. At present, second-generation sequencing technology platforms mainly include Hiseq, Miseq, Nextseq and Novaseq from Illumina, and SOLID system, PGM and Proton from Life Technologies, etc. The technical concept of second-generation sequencing technology is sequencing by synthesis, namely, a DNA sequence is determined according to signal changes caused by newly synthesized different bases. For example, the Illumina sequencing platform detects light signals, and the Life sequencing platform detects current changes caused by acid-base changes. So far, second-generation sequencing technology is the most mature and most extensively used DNA high-throughput sequencing method and plays an important role in large-scale genome sequencing and gene diagnosis and therapy. The clinical application thereof has also become more and more widespread.

Circulating DNA is also known as free DNA, and is the DNA that exists outside a cell in blood. The main source of free DNA is apoptotic cells or bone marrow cells. After being cleaved by nuclease *in vivo*, DNA released from these cells produces small fragments of DNA with a length of about 166 bp (Y. M. Dennis Lo, et al., Science Translational Medicine, 2010, 10: 61ra91). Free DNA is in the state of dynamic equilibrium *in vivo*, therefore, free DNA can be used as an important parameter for health assessment. Events such as tumorigenesis and organ transplantation can cause changes in the properties of free DNA in peripheral blood; these properties include the length of free DNA, base information, epigenetic modification, etc.; therefore, free DNA can be used as an important marker for non-invasive detection for the early diagnosis, monitoring and prognosis evaluation of diseases.

At present, the clinical application of non-invasive prenatal diagnosis by using free DNA as a molecular marker has obtained approval in all aspects, and many countries have comprehensively promoted the application of this technology. In addition to base information, the length information of free DNA is also a very important molecular marker. Studies have found that the nucleosomes, transcription factors, or DNA-binding proteins of different tissues or cells in different states can bind to different regions of DNA, which finally leads to changes in the length of free DNA and sequencing coverage. The source of these pieces of free DNA can be traced according to these differences, which brings about the dawn of a new era in the fields of the early diagnosis of cancer, organ transplantation and monitoring, etc. (Matthew W. Snyder et al., Cell, 2016, 1: 57-68). In addition, studies on tumor methylation that use high-throughput sequencing methods have found that DNA methylation difference signals between tumors and normal tissues are analyzed by using methylation sequencing, and this difference can be used to realize the early diagnosis of cancers. In addition, this difference, combined with specific methylation signals of different tissues, can also locate the specific location of tumors, which is of great significance for the diagnosis and treatment of cancers after early screening (Kun Sun et al., 2015, 5: 5503-12; Shicheng Guo et al., 2017., 3: 635-642).

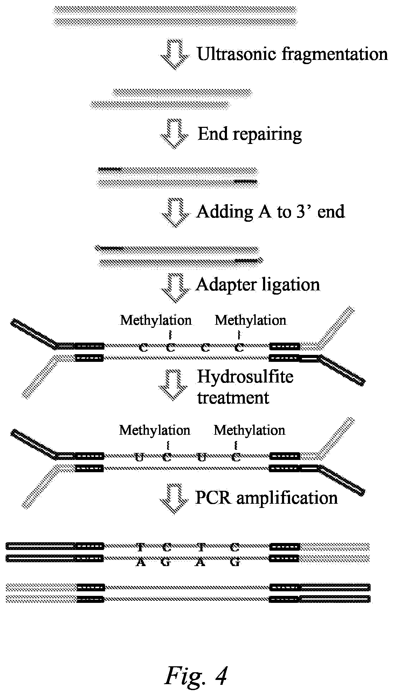

It is necessary to firstly construct a methylation sequencing library before using second-generation high-throughput sequencing technology for the methylation sequencing of free DNA. At present, the traditional construction process for a second-generation high-throughput methylation sequencing library (see figure 4) comprises firstly constructing a pre-library, which includes the steps of filling a terminus, phosphorylating a 5' terminus, overhanging A at a 3' terminus and ligation with an adapter; after the pre-library is constructed, the pre-library is treated with hydrosulfite, which can cause a large amount of DNA damage, and finally, the proportion of the template that can be sequenced is less than 10% of the original template (Masahiko Shiraishi et al., 2004, 10: 409-415). The process for constructing the methylation sequencing library requires: 1) each step requires purification, and the operation is complicated; 2) the filling step can artificially introduce nucleotides and change the true methylation status; 3) a large amount of a DNA template is destroyed during hydrosulfite treatment and lost after PCR amplification.

At present, the Swift methylation sequencing library construction method can be used to construct a library more efficiently than traditional methods (CN 104395480, see figure 5). The construction process comprises firstly performing a hydrosulfite treatment, and then constructing the library, including 3' terminus tailing and adapter ligation, then conducting an extension reaction, and then performing sequencing adapter ligation at the other end on the basis of obtaining a double-stranded deoxypolynucleotide. As the DNA template contains a large amount of dUTP during the extension reaction, the DNA template is damaged by the hydrosulfite treatment, and only one extension reaction is performed, the efficiency of obtaining complete double-stranded deoxypolynucleotide is low, there are fewer templates that can be used for ligation with the sequencing adapter at the other end, and finally, there are few templates that can be sequenced. In the field of genetic diagnosis, it has always been necessary to develop a better and more efficient method for constructing a library to improve the efficiency of template utilization.

### Summary of the Invention

In view of the current problems encountered in the construction of a DNA methylation sequencing library on the basis of a hydrosulfite treatment, the present invention provides a method for the adapter ligation of a single-stranded deoxypolynucleotide. Further provided is a method for constructing a second-generation high-throughput sequencing library. The method of the present invention is applicable not only to normal DNA, but also to severely damaged samples such as FFPE samples, ancient DNA, and DNA samples treated with hydrosulfite.

The present invention relates to a method for constructing a library of a deoxypolynucleotide substrate, the method comprising the following steps:
(1) mixing a single strand of the deoxypolynucleotide substrate with the following substances to form a first mixture: a) a deoxynucleotide selected from one of dGTP, dCTP, dATP and dTTP; b) a terminal deoxynucleotidyl transferase and a DNA ligase; c) a tail-controlling component, wherein the tail-controlling component is a partially double-stranded nucleotide molecule composed of a polynucleotide homopolymer with a length of 5 to 20 nucleotides, and an X region, and a linker polynucleotide complementary to the X region, wherein the polynucleotide homopolymer is complementary to the deoxynucleotide in a);
(2) incubating the first mixture, wherein the 3' end of the single strand of the deoxypolynucleotide substrate undergoes a tailing reaction with the deoxynucleotides in a solution, and the 3' end of the substrate, to which the homopolymeric polynucleotide tail has been added, is ligated to the linker of the tail-controlling component to obtain a tailed substrate;
(3) adding a DNA polymerase, deoxynucleotides comprising dGTP, dCTP, dATP and dTTP, and a linear amplification primer to the reaction system of step (2) to form a second mixture;
(4) incubating the second mixture, wherein the tailed substrate obtained from step (2) is used as a template to carry out a first linear extension reaction, a complementary strand of the substrate is synthesized and then melted, after the linear amplification primer is complementary to the substrate, a subsequent linear extension reaction is performed again, and the number of instances of linear extension reaction is not less than 3;
(5) melting the product of step (4);
(6) adding a 5' sequencing adapter and a DNA ligase to the solution of step (5) to form a third mixture;
(7) incubating the third mixture, wherein the 5' sequencing adapter is connected to the complementary strand of the substrate to prepare and obtain a DNA library.

In a specific embodiment, in the first linear extension reaction of step (4), the primer used is a tail-controlling molecule (that is, a single strand composed of a tail-controlling region and the X region), and in a subsequent extension reaction, the primer used is the linear amplification primer added in step (3).

In a specific embodiment, fragments of the polynucleotide homopolymer and X region in the tail-controlling component are degraded before the extension reaction of step (4) starts, and the linear extension reaction is carried out on the substrate by using the linear amplification primer added in step (3).

In a specific embodiment, the linear amplification primer added in step (3) competitively binds to the substrate, and thus, the linear extension reaction is carried out on the substrate by using the added linear amplification primer.

The present invention further relates to a kit, which can be used to construct a library of a deoxypolynucleotide substrate, and comprises:
a first component, comprising a deoxynucleotide selected from one of dGTP, dCTP, dATP and dTTP, a terminal deoxynucleotidyl transferase, a DNA ligase and a tail-controlling component, wherein the tail-controlling component is a partially double-stranded nucleotide molecule composed of a polynucleotide homopolymer with a length of 5 to 20 nucleotides, and an X region, and a linker polynucleotide complementary to the X region, and the polynucleotide homopolymer is complementary to the deoxynucleotide selected from one of dGTP, dCTP, dATP and dGTP;
a second component, comprising a DNA polymerase, deoxynucleotides comprising dGTP, dCTP, dATP and dTTP, and a linear amplification primer;
and a third component, comprising a 5' sequencing adapter and a DNA ligase.

In the present invention, by means of designing the linear amplification, the number of complementary strands of an original single-stranded polynucleotide substrate can be effectively increased. A 5' sequencing adapter can be efficiently added to the 3' terminus of a complementary strand of the polynucleotide substrate by designing a 5' sequencing adapter with several overhanging random bases at 3' terminus of one strand. Further, the polynucleotide substrate is denatured into a single strand, which, after the tailing of the substrate, is ligated to the linker; the linear amplification of the polynucleotide substrate is then completed to obtain a complementary strand; the 3' end of the complementary strand is ligated to the 5' sequencing adapter; PCR enrichment is then performed to obtain a library which can carry out next-generation sequencing.

In addition, since the substrate is methylated, a deoxypolynucleotide substrate template containing a U base is obtained. The U base may cause the linear amplification to stop, therefore, the obtained complementary strands vary in length. However, in the present invention, the 5' sequencing adapter with several random bases overhanging at the 3' terminus of one strand is ligated to the complementary strand, the single-stranded polynucleotide, which greatly improves the utilization rate of the complementary strand. According to the present invention, the present library construction process can be used to construct a whole genome methylation sequencing library from as little as 2 ng of genomic DNA derived from cultured human cells, and obtain efficient sequencing results.

### Brief Description of the Drawings

Figure 1: DNA library construction flowchart of the method of the present invention
Figure 2: Structure of a tail-controlling component
Figure 3: Structure of "5' sequencing adapter" with 6 overhanging Ns
Figure 4: DNA methylation library construction flowchart of a traditional method
Figure 5: DNA methylation library construction flowchart of the Swift method

### Detailed Description of the Invention

In the description, the terms 3', 3' end and 3' terminus have the same meaning, and the terms 5', 5' end and 5' terminus have the same meaning, and refer to the 3' end or 5' end of a nucleotide sequence, respectively.

### Polynucleotide substrate

The polynucleotide substrate is a polynucleotide substrate fragment that requires a tailing reaction and library construction. In various embodiments, the polynucleotide substrate is single-stranded or double-stranded DNA. In additional embodiments, the polynucleotide substrate is a chemically treated nucleotide sequence, including, but not limited to, polynucleotide that has been treated with hydrosulfite.

The polynucleotide substrate can be of natural origin or synthetic. The polynucleotide substrate of natural origin is a polynucleotide sequence derived from prokaryotes or eukaryotes, such as, derived from human, mouse, virus, plant, or bacterium. The polynucleotide substrate of the present invention may also be a severely damaged sample, such as an FFPE sample, ancient DNA, and a DNA sample treated with hydrosulfite. The polynucleotide substrate is tailed and can be used in assays involving microarrays and to generate a library for next-generation nucleic acid sequencing. The tailed polynucleotide substrate can also be used in the efficient cloning of the polynucleotide sequence.

In some embodiments, the polynucleotide substrate is single-stranded or double-stranded and comprises a 3'-end free hydroxyl group. In some aspects, the polynucleotide substrate is double-stranded and comprises a blunt terminus. In other aspects, the double-stranded polynucleotide substrate comprises a 3' recessed terminus. The length of a protruding terminus or recessed terminus of the polynucleotide substrate can vary. In various aspects, the length of the protruding terminus or recessed terminus of the polynucleotide substrate is 1, 2, 3, 4, 5, 6, 7, 8, 9, 10 or more nucleotides.

In some aspects, the length of the polynucleotide substrate is between about 10 and about 5000 nucleotides, or between about 40 and about 2000 nucleotides, or between about 50 and about 1000 nucleotides, or between about 100 and about 500 nucleotides. In additional aspects, the length of the polynucleotide substrate is at least 3 to at most about 50, 100 or 1000 nucleotides.

### DNA dephosphorylation

DNA dephosphorylation refers to the removal of phosphate groups of first amino acid residues at the 5' end and 3' end of DNA; generally, alkaline phosphatase is used to treat the DNA to achieve the dephosphorylation of the 5'-end and 3'-end residues.

In some embodiments, in the present invention, the dephosphorylation reaction for the deoxypolynucleotide substrate is performed before the deoxypolynucleotide substrate is tailed.

### Tailing reaction

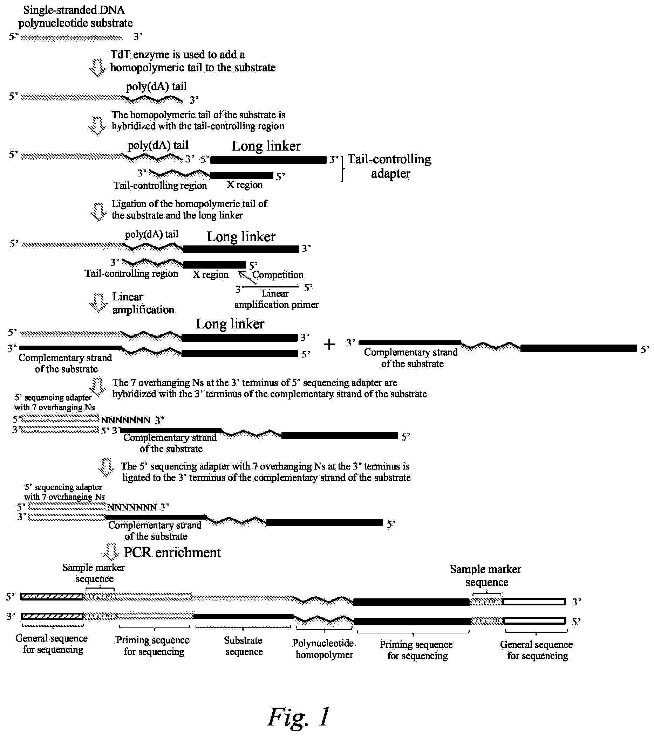

The term "tailing" as used herein is interchangeable with the term "controlled tailing". In step (2) of the method of the present invention, tailing is carried out on the deoxypolynucleotide substrate, and the method is used to add a required number of nucleotides to the 3' end of the polynucleotide substrate in a controlled manner. By way of example and in a non-limiting manner, the tail of the polynucleotide substrate is controlled within a certain length range by adding the tail-controlling component (see figure 1). The tail-controlling component comprises a polynucleotide homopolymer with a length of 5 to 20 nucleotides, and the tail-controlling component and the newly added homopolymeric nucleotide tail sequence of the substrate form a double-stranded structure, thus, the speed of the polymerization process is reduced and the tail of the polynucleotide substrate is controlled within a certain length range (see figure 1).

The nucleotide added in the tailing reaction is a deoxynucleotide selected from one of dGTP, dCTP, dATP and dTTP, and may be, for example a dGTP solution, or a dCTP solution, or a dATP solution, or a dTTP solution. In one specific embodiment, a tail-controlling component containing a poly (dT) homopolymeric nucleotide sequence is used to control the TdT enzyme (terminal deoxynucleotidyl transferase) to add a poly (dA) tail (also known as a homopolymeric nucleotide (dA) tail) to the 3' end of the polynucleotide substrate. Further, the homopolymeric tail of the polynucleotide substrate is ligated to a linker of the tail-controlling component to form a 3'-end tailing region of the substrate.

In one specific embodiment, the tail-controlling component comprises the homopolymer nucleotide sequence with 5-20, preferably 5-13, further preferably 7-10, and more preferably 7-9 identical nucleotides.

In one specific embodiment, the molar concentration ratio of the polynucleotide substrate to the tail-controlling component ranges from 1 : 1-1 : 100, and preferably 1 : 5-1 : 50.

In one specific embodiment, the pH of the tailing reaction in step (2) of the method of the present invention ranges from about 5.0 to about 9.0; the molar concentration ratio of the polynucleotide substrate to the single nucleotide ranges from 1 : 10-1 : 20,000, and preferably 1 : 100-1 : 2000; the incubation time is 1 minute to 120 minutes, preferably 0.5-60 minutes, 0.5-30 minutes, 1-20 minutes, 1-15 minutes or 1-10 minutes; the incubation temperature is 20°C-50°C, preferably 25°C-45°C, and more preferably 25°C-37°C.

### Tail-controlling component

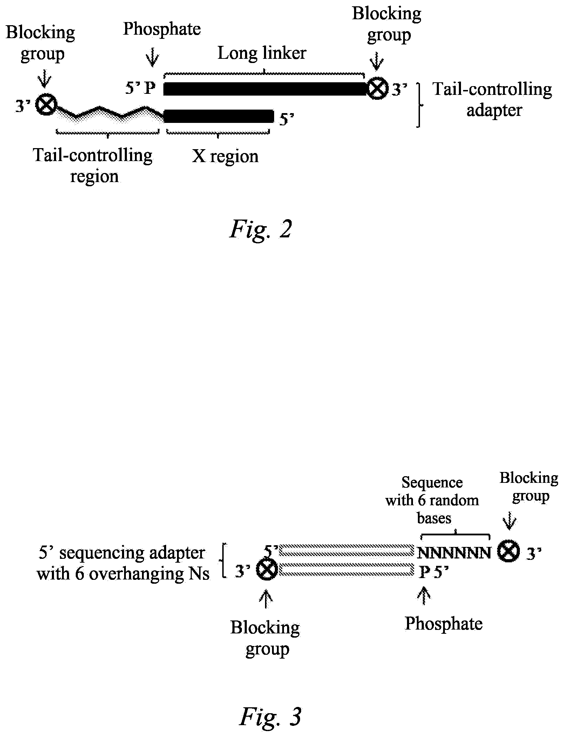

In the present invention, the tailing length and efficiency of the polynucleotide substrate are controlled by adding a tail-controlling component. The tail-controlling component is composed of a tail-controlling region and an X region, and a linker sequence that can be complementary to the X region (see figure 2). The tail-controlling component is also known as a "tail-controlling adapter". The single strand composed of the tail-controlling region and the X region is referred to as a "tail-controlling molecule".

The tail-controlling region of the present invention is a polynucleotide homopolymer with a length of 5-20 nucleotides. The polynucleotide homopolymer, also known as a "poly region", is a polynucleotide strand formed by ligating identical nucleotides. The tail-controlling region of the present invention is a homopolymer nucleotide sequence composed of a deoxynucleotide of one of dGTP, dCTP, dATP and dTTP; preferably, the tail-controlling region is a poly (dT) tail-controlling region composed of dTTP. Preferably, the length of the polynucleotide homopolymer of the tail-controlling region of the present invention is 5-20 nucleotides, preferably 7-20, 9-20 nucleotides, and further preferably 5-10 nucleotides, 7-10 nucleotides, and more preferably 7-9 nucleotides. The dGTP or dCTP polynucleotide homopolymer with a certain length can effectively control the tailing for the polynucleotide substrate to be about 20 nucleotides.

The "X region sequence" provides a priming sequence for the amplification or sequencing of nucleic acid fragments, and can also comprise a marker sequence for distinguishing different substrate molecules. The marker sequence can comprise 4-16 bases, and is used for next-generation sequencing applications in some aspects. In some embodiments of the present invention, the X region sequence can be, but is not limited to, a next-generation sequencing (NGS) adapter sequence which is compatible with an Illumina, Ion Torrent, Roche 454 or SOLiD sequencing platform. The X region sequence can be a DNA sequence, an RNA sequence, or a heteropolymeric sequence comprising DNA and RNA.

For the linker in the tail-controlling component, only the linker that is complementary to the X region sequence is referred to as a "short linker"; a linker sequence that includes an extended primer binding region in addition to the sequence complementary to the X region is referred to as a "long linker", and is as shown in Table 2.

In the present invention, a method for deoxypolynucleotide substrate tailing is used, wherein the method is used to add a required number of nucleotides to the 3' end of the polynucleotide substrate in a controlled manner. By way of example and in a non-limiting manner, by means of adding the tail-controlling component, wherein the tail-controlling component comprises a polynucleotide homopolymer with a length of 5-20 nucleotides, and the tail-controlling component is complementary to the newly added homopolymeric nucleotide tail sequence of the substrate, thereby forming a double-stranded structure, thus reducing the speed of the polymerization process, the tail of the polynucleotide substrate is controlled within a certain length range (see figure 1).

In some embodiments of the present invention, a tail-controlling component containing a poly(dT) homopolymeric nucleotide sequence is used to control the TdT enzyme to add a poly(dA) tail (also known as a homopolymeric nucleotide (dA) tail) to the 3' end of the polynucleotide substrate. Further, the poly (dA) tail of the polynucleotide substrate is ligated to the linker of the tail-controlling component to form the 3'-end tailing region of the substrate.

In some embodiments of the present invention, the tail-controlling component comprises a blocking group. The blocking group used herein is a part that prevents extension by means of an enzyme. If there is no blocking group, the enzyme can synthesize the polynucleotide by adding nucleotides. The blocking group includes, but is not limited to, a phosphate group, a carbon 3 spacer, a dideoxynucleotide, a ribonucleotide, an amino and reverse deoxythymidine.

In some embodiments of the present invention, the linker of the tail-controlling component has a phosphorylation modification at the 5' end and a blocking group at the 3' end; the tail-controlling region has a blocking group at 3'.

### Linear amplification reaction

A linear amplification reaction is also known as a "linear extension reaction" or "linear extension".

In the present invention, after the deoxypolynucleotide substrate is tailed, the deoxypolynucleotide substrate is further used as a template to perform a linear amplification reaction. The present invention provides a method for the linear amplification of the deoxynucleotide substrate, and the method is used to increase the amount of deoxypolynucleotide substrates in a linear amplification manner (see figure 1).

In some embodiments of the present invention, after the tailing reaction, a linear amplification primer is added; the tailed substrate is used as a template to carry out the extension reaction; the extension reaction may first be an extension reaction that takes place by means of a tail-controlling molecule. In some embodiments of the present invention, the linear amplification primer separates the tail-controlling molecule from the substrate polynucleotide substrate by means of competition, and then, the deoxypolynucleotide substrate is used as a template to perform the linear amplification reaction. In some specific embodiments, fragments of the polynucleotide homopolymer and X region in the tail-controlling component are degraded before the extension reaction of step (4) starts, and the linear extension reaction is carried out on the substrate by using the linear amplification primer added in step (3).

In a specific embodiment, the method comprises: after the tailing reaction, the polynucleotide substrate being denatured to a single-stranded state, and adding the linear amplification primer complementary to the 3' linker sequence of the substrate, DNA polymerase and deoxynucleotides to react with the polynucleotide substrate. a nucleotide extension reaction occurring at the 3' end of the linear amplification primer to synthesize the complementary strand of the substrate and obtain a double-stranded deoxypolynucleotide; denaturing the double-stranded deoxypolynucleotide to separate the complementary strand of the substrate from the substrate; the substrate again undergoing an extension reaction with the linear amplification primer, the DNA polymerase and deoxynucleotides, wherein the number of times the extension reaction takes place is referred to as the number of linear amplification cycles. In some aspects, the DNA polymerase can efficiently amplify a deoxypolynucleotide substrate template containing a U base.

In a specific embodiment, the number of linear amplification cycles is not less than 3, preferably not less than 4, preferably 4-50, further preferably 4-20 and more preferably 4-12 and 8-12.

### Ligation reaction of the 5' sequencing adapter

The 5' sequencing adapter of the present invention is a deoxypolynucleotide with a partially double-stranded structure; "partially double-stranded" refers to a 5' sequencing adapter with a random number of overhanging bases comprising a single-stranded part and a double-stranded part.

The 5' sequencing adapter provides a priming sequence for the amplification or sequencing of nucleic acid fragments, and is used for next-generation sequencing applications in some aspects.

In the present invention, the 5' sequencing adapter with overhanging random bases undergoes a ligation reaction with the complementary strand of the substrate obtained after the linear amplification reaction, and the 5' sequencing adapter with overhanging random bases comprises a single-stranded polynucleotide of random bases at the 3' terminus (see figure 3), therefore, the 5' sequencing adapter with overhanging random bases has a partially double-stranded polynucleotide and is of a multi-molecular structure, and the 5' sequencing adapter with overhanging random bases is also referred to as "5' sequencing adapter with overhanging Ns". For example, a 5' sequencing adapter with 6 overhanging random bases is also referred to as a "5' sequencing adapter with 6 overhanging Ns", wherein N represents a deoxynucleotide base, that is to say, each of the 6 Ns is a deoxynucleotide base randomly selected from overhanging dGTP, dCTP, dATP and dTTP. The "5' sequencing adapter with 6 overhanging Ns" is a mixed molecule by ligating different random bases.

The number of overhanging random bases in the 5' sequencing adapter of the present invention is 0-50, preferably 2-30, further preferably 2-17, 4-15 and 4-10, and more preferably 7-10 and 7-9.

In a specific embodiment of the present invention, the 5' sequencing adapter is formed by annealing two polynucleotide strands. A polynucleotide strand without random bases has a phosphorylation modification at the 5' end, and a blocking group at the 3' end; a polynucleotide strand containing random bases has a blocking group at the 3' end (see figure 3).

In a method for ligating an adapter to a single-stranded deoxypolynucleotide, the method is used for the complementing of the 3' end of the complementary strand of the substrate obtained after the linear amplification reaction by the random base single-stranded polynucleotide portion of the 5' sequencing adapter with overhanging random bases to obtain a partially double-stranded structure in addition to the double-stranded portion of the 5' sequencing adapter. Under the effect of the DNA ligase, the 5' end of the polynucleotide strand not containing random bases of the 5' sequencing adapter is ligated to the 3' end of the complementary strand of the polynucleotide substrate (see figure 1). After the linear amplification step and a purification step, the complementary strands of the substrate used to ligate with the 5' sequencing adapter are increased, and ligation efficiency is improved.

In a specific embodiment, the molar concentration ratio of the polynucleotide substrate to the 5' sequencing adapter ranges from 1 : 100-1 : 4000, preferably 1 : 500-1 : 1000.

### Ligase

The ligase that can be used in the method of the present invention can be DNA ligase and RNA ligase, including, but not limited to, T4 DNA ligase, *Escherichia coli* DNA ligase, T7 DNA ligase and T4 RNA ligase.

The ligase of the present invention ligates the linker in the tail-controlling component with tailed polynucleotide of the substrate. In other embodiments, the ligase of the present invention ligates the 5' sequencing adapter to the synthesized single-stranded deoxypolynucleotide, which is the complementary strand of the substrate.

### Separation step

In some embodiments, the polynucleotide product after step (7) of the present invention is purified. The purification of the polynucleotide product is carried out by any method known to and understood by those skilled in the art. The polynucleotide substrate of the present invention can be purified by adding magnetic beads with carboxy modified surfaces. In other specific embodiments, the polynucleotide substrate is purified by means of column purification and precipitation.

### Detailed Description of Embodiments

For the sequences used in the examples, please see Table 1-Table 6, which follow:

**Table 1. DNA polynucleotides for the examples**

| Serial number | Sequence (direction: 5'-3') | Description | Serial number in the sequence listing |
|---|---|---|---|
| 001 | AGACGTGTGCTCTTCCGATCTTTTTTTTTT*T*T*T -C3 Spacer | Tail-controlling molecule having a 12b poly(dT) tail-controlling region | SEQ ID NO: 1 |
| 002 | Phos-AGATCGGAAGAGCACACGTCTGAACTCCAGT*C*A* C-C3 Spacer | Long linker | SEQ ID NO: 2 |
| 003 | GTGACTGGAGTTCAGACGTGTGCTCTTCCGATCT | Linear amplification primer | SEQ ID NO: 3 |
| 004 | GTGACTGGAGTTCAGACGTGT | qPCR reverse primer | SEQ ID NO: 4 |
| 005 | ACACTCTTTCCCTACACGACG | qPCR forward primer | SEQ ID NO: 5 |
| 006 | Phos-NNNNNNNNAGATCGGAAGAGCACACGTCTGAACT CCAGT*C*A*C-C3 Spacer | Long linker with tag | SEQ ID NO: 6 |
| 007 | ACACTCTTTCCCTACACGACGCTCTTCCGATC*T*N*N-C3 Spacer | 5' sequencing adapter forward primer containing 2 random bases at the 3' terminus | SEQ ID NO: 7 |
| 008 | ACACTCTTTCCCTACACGACGCTCTTCCGATCT*N*N*N-C 3 Spacer | 5' sequencing adapter forward primer containing 3 random bases at the 3' terminus | SEQ ID NO: 8 |
| 009 | ACACTCTTTCCCTACACGACGCTCTTCCGATCTN*N*N*N -C3 Spacer | 5' sequencing adapter forward primer containing 4 random bases at the 3' terminus | SEQ ID NO: 9 |
| 010 | ACACTCTTTCCCTACACGACGCTCTTCCGATCTNN*N*N* N-C3 Spacer | 5' sequencing adapter forward primer containing 5 random bases at the 3' terminus | SEQ ID NO: 10 |
| 011 | ACACTCTTTCCCTACACGACGCTCTTCCGATCTNNN*N*N *N-C3 Spacer | 5' sequencing adapter forward primer containing 6 random bases at the 3' terminus | SEQ ID NO: 11 |
| 012 | ACACTCTTTCCCTACACGACGCTCTTCCGATCTNNNN*N* N*N-C3 Spacer | 5' sequencing adapter forward primer containing 7 random bases at the 3' terminus | SEQ ID NO: 12 |
| 013 | ACACTCTTTCCCTACACGACGCTCTTCCGATCTNNNNN* N*N*N-C3 Spacer | 5' sequencing adapter forward primer containing 8 random bases at the 3' terminus | SEQ ID NO: 13 |
| 014 | ACACTCTTTCCCTACACGACGCTCTTCCGATCTNNNNNN *N*N*N-C3 Spacer | 5' sequencing adapter forward primer containing 9 random bases at the 3' terminus | SEQ ID NO: 14 |
| 015 | Phos-AGATCGGAAGAGCGTCGTGTAGGGAAAGAG*T*G* T-C3 Spacer | 5' sequencing adapter reverse primer | SEQ ID NO: 15 |
| 016 | A/5mC/A/5mC/T/5mC/TTT/5mC//5mC//5mC/TA/5mC/A/5mC/ GA/5mC/G/5mC/T/5mC/TT/5mC//5mC/GAT/5mC/T | Traditional methylation sequencing adapter forward primer | SEQ ID NO: 16 |
| 017 | Phos-GAT/5mC/GGAAGAG/5mC/A/5mC/A/5mC/GT/5mC/TG AA/5mC/T/5mC//5mC/AGT/5mC/A/5mC/ | Traditional methylation sequencing adapter reverse primer | SEQ ID NO: 17 |
| 018 | AATGATACGGCGACCACCGAGATCTACACACAGTGCTA CACTCTTTCCCTACACGACG | P5 PCR label primer M501 | SEQ ID NO: 18 |
| 019 | AATGATACGGCGACCACCGAGATCTACACCGCATGTAA CACTCTTTCCCTACACGACG | P5 PCR label primer M502 | SEQ ID NO: 19 |
| 020 | AATGATACGGCGACCACCGAGATCTACACTATCGCAGA CACTCTTTCCCTACACGACG | P5 PCR label primer M503 | SEQ ID NO: 20 |
| 021 | AATGATACGGCGACCACCGAGATCTACACGTGCATACA CACTCTTTCCCTACACGACG | P5 PCR label primer M504 | SEQ ID NO: 21 |
| 022 | AATGATACGGCGACCACCGAGATCTACACAGCTAGTCA CACTCTTTCCCTACACGACG | P5 PCR label primer M505 | SEQ ID NO: 22 |
| 023 | AATGATACGGCGACCACCGAGATCTACACCTAGCTGAA CACTCTTTCCCTACACGACG | P5 PCR label primer M506 | SEQ ID NO: 23 |
| 024 | CAAGCAGAAGACGGCATACGAGATGTCTTAGGGTGACT GGAGTTCAGACG | P7 PCR label primer N726 | SEQ ID NO: 24 |
| 025 | CAAGCAGAAGACGGCATACGAGATTAGCTGCAGTGACT GGAGTTCAGACG | P7 PCR label primer N728 | SEQ ID NO: 25 |
| 026 | AATGATACGGCGACCACCGAGATCTACACTCTTTCCCTA CACG*A*C | P5 PCR primer | SEQ ID NO: 26 |
| 027 | CAAGCAGAAGACGGCATACGAGATTTGACTGTGACTGG AGTTCAGA*C*G | P7 PCR label primer 13 | SEQ ID NO: 27 |
| 028 | CAAGCAGAAGACGGCATACGAGATTGACATGTGACTGG AGTTCAGA*C*G | P7 PCR label primer 14 | SEQ ID NO: 28 |
| 029 | AGACGTGTGCTCTTCCGATCNNNNNNNNTTTTTTTTTTT*T *T*T-C3 Spacer | Tagged tail-controlling molecule having a 12b poly(dT) tail-controlling region | SEQ ID NO: 29 |

| | | | |
|---|---|---|---|
| Phos: Phosphate; *: Thio site; C3 Spacer: Carbon 3 Spacer; 5mC: 5-methyl-cytosine deoxynucleotide; N: dA, dT, dC or dG nucleotide | | | |

**Table 2. Tail-controlling component with overhanging poly(dT) for constructing a methylation sequencing library**

| **Serial number of polynucleotide** | **Structure of adapter** |
|---|---|
| 002 | Long linker /5'Phos/AGATCGGAAGAGCACACGTCTGAACTCCAGT*C*A*C /3' C3 Spacer Tail-controlling /3' C3 Spacer/T*T*T*TTTTTTTTTCTAGCCTTCTCGTGTGCAGA 5' molecule tail-controlling region X region |
| 001 | |

| | |
|---|---|
| Phos: Phosphate; C3 Spacer: Carbon 3 Spacer; *: Thio site | |

**Table 3. 5' sequencing adapters with different numbers of overhanging Ns for example 1**

| **Serial number of polynucleotide** | **Structure of adapter** |
|---|---|
| 012 | 5' sequencing- adapter with 7 5'ACACTCTTTCCCTACACGACGCTCTTCCGATCTNNNN*N*N*N/3' C3 Spacer/ overhanging Ns /3' C3 Spacer/T*G*T*GAGAAAGGGATGTGCTGCGAGAAGGCTAGA/5'Phos/ |
| 015 | |
| 007 | 5' sequencing-    5'ACACTCTTTCCCTACACGACGCTCTTCCGATC*T*N*N/3'  C3 Spacer/ adapter with 2 overhanging Ns    /3' C3 Spacer/T*G*T*GAGAAAGGGATGTGCTGCGAGAAGGCTAGA/5'Phos/ |
| 015 | |
| 008 | 5' sequencing-    5'ACACTCTTTCCCTACACGACGCTCTTCCGATCT*N*N*N/3' C3 Spacer/ adapter with 3    /3' C3 Spacer/T*G*T*GAGAAAGGGATGTGCTGCGAGAAGGCTAGA/5'Phos/ overhanging Ns |
| 015 | |
| 009 | 5' sequencing-          5'ACACTCTTTCCCTACACGACGCTCTTCCGATCTN*N*N*N/3'  C3 Spacer/ adapter with 4   /3' C3 Spacer/T*G*T*GAGAAAGGGATGTGCTGCGAGAAGGCTAGA/5'Phos/ overhanging Ns |
| 015 | |
| 010 | 5' sequencing- 5'ACACTCTTTCCCTACACGACGCTCTTCCGATCTNN*N*N*N/3' C3 Spacer/ adapter with 5 /3' C3 Spacer/T*G*T*GAGAAAGGATGTGCTGCGAGAAGGCTAGA/5'Phos/ overhanging Ns |
| 015 | |
| 011 | 5' sequencing- 5'ACACTCTTTCCCTACACGACGCTCTTCCGATCTNNN*N*N*N/3' C3 Spacer/ adapter with 6 /3' C3 Spacer/T*G*T*GAGAAAGGGATGTGCTGCGAGAAGGCTAGA/5'Phos/ overhanging Ns |
| 015 | |
| 013 | 5' sequencing- 5'ACACTCTTTCCCTACACGACGCTCTTCCGATCTNNNNN*N*N*N/3' C3 Spacer/ adapter with 8 /3' C3 Spacer/T*G*T*GAGAAAGGGATGTGCTGCGAGAAGGCTAGA/5'Phos/ overhanging Ns |
| 015 | |
| 014 | 5' sequencing- adapter with 9 overhanging Ns 5'ACACTCTTTCCCTACACGACGCTCTTCCGATCTNNNNNN*N*N*N/3' C3 Spacer/ /3' C3 Spacer/T*G*T*GAGAAAGGGATGTGCTGCGAGAAGGCTAGA/5'Phos/ |
| 015 | |

| | |
|---|---|
| Phos: Phosphate; C3 Spacer: Carbon 3 Spacer; *: Thio site; N: dA, dT, dC or dG nucleotide | |

**Table 4. Tail-controlling component with overhanging poly (dT) and molecular tag for constructing a methylation sequencing library**

| **Serial number of polynucleotide** | **Structure of adapter** |
|---|---|
| 006 | Long linker with tag /5' Phos/NNNNNNNNAGATCGGAAGAGCACACGTCTGAACTCCAGT*C*A*C /3' C3 Spacer Tail-controlling molecule with tag /3' C3 Spacer/T*T*T*TTTTTTTTTTNNNNNNNNTCTAGCCTTCTCGTGTGCAGA 5' tail-controlling region X region |
| 029 | |

| | |
|---|---|
| Phos: Phosphate; C3 Spacer: Carbon 3 Spacer; *: Thio site; N: dA, dT, dC or dG nucleotide | |

**Table 5. "Traditional methylation sequencing adapter" for constructing a methylation sequencing library**

| **Serial number of polynucleotide** | **Structure of adapter** |
|---|---|
| 016 | 5' A/5mC/A/5mC/T/5mC/TTT/5mC//5mC//5mC/TA/5mC/A/5mC/GA/5mC/G/5mC/T/5mC/TT/5mC//5mC/GAT/5mC/T 3' /5mC/A/5mC/TGA/5mC//5mC/T/5mC/AAGT/5mC/TG/5mC/A/5mC/A/5mC/GAGAAGG/5mC/TAG/5' Phos/ |
| 017 | |

| | |
|---|---|
| Phos: Phosphate; 5mC: 5-methyl-cytosine deoxynucleotide | |

### EXAMPLES

### Example 1. The effect of 5' sequencing adapters with different numbers of overhanging random bases on the ligation of 5' sequencing adapter

### Materials:

5 x annealing buffer (Beyotime, Catalog No. D0251)
Enzyme-free water (Solarbio, Catalog No. R1600-100)
λ-DNA (Takara, Catalog No. 3019)
Hydrosulfite treatment kit (Zymo Research, Catalog No. D5005)
FastAP thermo-sensitive alkaline phosphatase (ThermoFisher, EF0651, 1 U/µL)
10 x CutSmart buffer (New England Biolabs, Catalog No. B7204S)
10 x green buffer (Enzymatics, Catalog No. B0120, 20 mM Tris-acetate, 50 mM potassium acetate and 10 mM magnesium acetate, pH 7.9)
β-nicotinamide adenine dinucleotide (New England Biolabs, Catalog No. B9007S, 50 mM)
dATP (Takara, Catalog No. 4026, 100 mM)
TdT enzyme (Enzymatics, Catalog No. P7070L, 20 U/µL)
*Escherichia coli* DNA ligase (Takara, Catalog No. 2161, 60 U/µL)
EB buffer (Qiagen, Catalog No. 19086)
dNTP (Takara, Catalog No. 4030, 2.5 mM each)
Phusion U Hot Start DNA polymerase (ThermoFisher, Catalog No. F555L, 2 U/µL)
5 x Phusion HF buffer (ThermoFisher, Catalog No. F555L)
Beckman Ampure XP magnetic bead (Beckman, Catalog No. A63882)
SB buffer: 20% PEG8000, 2.5 M NaCl, 10 mM Tris-hydrochloric acid, 1mM EDTA
2 x T4 DNA quick ligation reaction buffer (Enzymatics, Catalog No. B1010)
T4 DNA quick ligase (Enzymatics, Catalog No. L6030-HC-L, 600 U/µl)

### Method:

(1) Adapter preparation: Polynucleotide pairs (001/002, 007/015, 008/015, 009/015, 010/015, 011/015, 012/015, 013/015, 014/015) were mixed in equimolar amounts, incubated in 1 x annealing buffer at 95°C for 2 minutes, then cooled slowly to room temperature to obtain the tail-controlling adapter (as shown in Table 2) and the 5' sequencing adapters with different numbers of overhanging random bases at the 3' terminus (as shown in Table 3).
(2) 40 ng of λ-DNA (Takara, Catalog No. 3019) was treated with hydrosulfite by using a hydrosulfite treatment kit.
(3) The DNA product of step (2) was fragmented to 300 bp by using a focused ultrasonic apparatus (Covaris, Catalog No. S220), and retained for use.
(4) A DNA dephosphorylation reaction mixture was prepared as shown in Table 1-1. The reaction mixture was placed in a warm bath at 37°C for 30 minutes, treated at 95°C for 5 minutes, then immediately inserted into ice and incubated for 2 minutes, then retained for use, so that the substrate remained in a single-stranded state.

**Table 1-1**

| DNA dephosphorylation reaction mixture | |
|---|---|
| DNA (from step (3)) | 17 µl |
| 10 x CutSmart buffer | 2 µl |
| FastAP thermo-sensitive alkaline phosphatase | 1 µl |
| Total volume | 20 µl |

(5) Tailing the polynucleotide substrate: A tailing and ligation reaction mixture was prepared as shown in Table 1-2. The reaction mixture was placed in a warm bath at 37°C for 30 minutes, treated at 95°C for 5 minutes, and then kept at 4°C.

**Table 1-2**

| Tailing and ligation reaction mixture | |
|---|---|
| DNA (from step (4)) | 20 µl |
| Enzyme-free water | 4 µl |
| 2.5 x green buffer plus 12.5% PEG 8000 | 8 µl |
| 2 mM dATP plus 2 mM β-nicotinamide adenine dinucleotide | 2 µl |
| 10 µM tail-controlling adapter (001/002 as shown in Table 2) | 2 µl |
| 1 : 33 diluted TdT enzyme (1 µl TdT enzyme plus 32 µl EB buffer) | 2 µl |
| 1 : 6 diluted *Escherichia coli* DNA ligase (1 µl TdT enzyme plus 5 µl EB buffer) | 2 µl |
| Total volume | 40 µl |

(6) Linear amplification: The reaction mixture for linear amplification as shown in Table 1-3 was prepared, and 4 linear amplifications were run according to the PCR amplification program as shown in Table 1-4. The linear amplification product was purified and recovered by using 166 µl of 1 : 6 diluted Beckman Ampure XP magnetic beads (1 volume of Beckman Ampure XP magnetic beads plus 5 volumes of SB buffer) and 280 µl of 1.8 : 1 diluted SB buffer (1.8 volume of SB buffer plus 1 volume of enzyme-free water), then eluted by adding 100 µl of EB buffer. 100 µl of eluent was divided into 5 µl/part, with each part being charged into a 200 µl PCR tube, and 18 parts were separated and used for a next-step reaction.

**Table 1-3**

| Reaction mixture for linear amplification | |
|---|---|
| DNA (from step (5)) | 40 µl |
| Enzyme-free water | 29 µl |
| 5 x Phusion HF buffer | 20 µl |
| 100 µM linear amplification primer (003) | 2 µl |
| 2.5 mM dNTP | 8 µl |
| Phusion U Hot Start DNA polymerase | 1 µl |
| Total volume | 100 µl |

**Table 1-4**

| PCR reaction condition | | |
|---|---|---|
| Temperature | Time | Number of cycles |
| 95°C | 3 minutes | 1 |
| 95°C | 30 seconds | 4 |
| 60°C | 30 seconds | |
| 68°C | 1 minute | |
| 68°C | 5 minutes | 1 |
| 4 | Preservation | 1 |

(7) The 18 parts of DNA in step (6) were reacted at 95°C for 5 minutes, and immediately placed on ice for 2 minutes and retained for use, so that the two strands were melted and maintained as a single strand for use.
(8) The ligation reaction mixture of the DNA from step (7) and a "5' sequencing adapter" with 2-9 overhanging Ns was prepared as shown in Table 1-5. Two of each type of adapter were in parallel. The reaction mixture was placed in a warm bath at 25°C for 15 minutes. The ligated DNA was recovered by using 17 µl of Beckman Ampure XP magnetic beads, and then eluted by adding 26 µl of enzyme-free water to obtain a PCR amplification pre-library.

**Table 1-5**

| "5' sequencing adapter" ligation reaction mixture | |
|---|---|
| DNA (from step (7)) | 5 µl |
| Enzyme-free water | 2 µl |
| 2 x T4 DNA quick ligation reaction buffer | 10 µl |
| 10 µM "5' sequencing adapter" with n overhanging Ns (Table 3) | 2 µl |
| T4 DNA quick ligase | 1 µl |
| Total volume | 20 µl |

(9) The molar concentration of the PCR amplification pre-library was detected by using a library quantification kit (KAPA Biosystems, Catalog No. KK4824) and a DNA quantification standard and premix primer kit (KAPA Biosystems, Catalog No. KK4808) plus a qPCR forward primer/qPCR reverse primer (005/004, as shown in Table 1, the use concentration thereof being the same as that of the premix primer kit), and during calculation, the size of the fragment of the PCR amplification pre-library was 320 bp.

Experiment results: As shown in able 1-6, the concentration of the PCR amplification pre-library constructed by using the "5' sequencing adapter" with 2 overhanging Ns was the lowest, at 0.000780 nM, and as the number of overhanging Ns in "5' sequencing adapter" increased from 2 to 4, the concentration of the PCR amplification pre-library increased from 0.000780 nM to 0.0254 nM, this being an exponential increase.T The increase of the concentration of the PCR amplification pre-library was weakened as the number of overhanging Ns in "5' sequencing adapter" increased from 4 to 7; and the concentration of the PCR amplification pre-library constructed by using the "5' sequencing adapter" with 7 overhanging Ns was the highest, at 0.0653 nM. The concentrations of the PCR amplification pre-libraries constructed by using the "5' sequencing adapters" with 7 overhanging Ns, 8 overhanging Ns and 9 overhanging Ns (see Table 3) were substantially the same, at 0.0653 nM, 0.0627 nM and 0.0646 nM, respectively.

Conclusion: All "5' sequencing adapters" with 2-9 overhanging Ns can effectively undergo a ligation reaction with the complementary strands of the nucleotide substrate obtained after linear amplification, and can be used to construct the methylation library for DNA treated with hydrosulfite.

**Table 1-6**

| Number of overhanging random bases (Ns) in "5' sequencing adapter" | 2 | 3 | 4 | 5 | 6 | 7 | 8 | 9 |
|---|---|---|---|---|---|---|---|---|
| Concentration of library (nM) | 0.000780 | 0.00503 | 0.0254 | 0.0404 | 0.0503 | 0.0653 | 0.0627 | 0.0646 |

### Example 2. The effect of different concentrations of the adapter on the ligation of 5' sequencing adapter

### Methods:

(1) Adapter preparation: A tail-controlling adapter (001/002, as shown in Table 2) and a "5' sequencing adapter" with 6 overhanging Ns at the 3' terminus (011/015, as shown in table 3) were prepared according to the adapter preparation method in example 1.
(2) 24 ng of λ-DNA was treated with hydrosulfite by using a hydrosulfite treatment kit.
(3) Preparation of linear amplification product: The linear amplification product was prepared according to the method described in example 1. During the purification and recovery of the linear amplification product, 31.2 µl of EB buffer was added for elution. 31.2 µl of the eluent was divided into 2.6 µl/part, with each part being charged into a 200 µl PCR tube, and 10 parts were separated and used for a next-step reaction.
(4) The DNA in step (3) was reacted at 95°C for 5 minutes, and immediately placed on ice for 2 minutes and retained for use.
(5) Reaction: The reaction mixture for the ligation of the "5' sequencing adapter" was prepared as shown in Table 2-1. The reaction mixture was placed in a warm bath at 25°C for 15 minutes. The ligated DNA was recovered by using 17 µl of Beckman Ampure XP magnetic beads, and then eluted by adding 26 µl of enzyme-free water to obtain a PCR amplification pre-library.

**Table 2-1**

| "5' sequencing adapter" ligation reaction mixture | | | | | |
|---|---|---|---|---|---|
| Proportion of DNA substrate: 5' sequencing adapter | 1 : 100 | 1 : 500 | 1 : 1000 | 1 : 2000 | 1 : 4000 |
| DNA (from step (4)) | 2.6 µl | 2.6 µl | 2.6 µl | 2.6 µl | 2.6 µl |
| Enzyme-free water | 6 µl | 4.4 µl | 2.4 µl | 3.2 µl | - |
| 2 x T4 DNA quick ligation reaction buffer | 10 µl | 10 µl | 10 µl | 10 µl | 10 µl |
| 10 µM "5' sequencing adapter" with 6 overhanging Ns | 0.4 µl | 2 µl | 4 µl | - | - |
| 25 µM "5' sequencing adapter" with 6 overhanging Ns | - | - | - | 3.2 µl | 6.4 µl |
| T4 DNA quick ligase | 1 µl | 1 µl | 1 µl | 1 µl | 1 µl |
| Total volume | 20 µl | 20 µl | 20 µl | 20 µl | 20 µl |

(6) The molar concentration of the PCR amplification pre-library was detected according to the method described in Example 1.

Experiment results: As shown in Table 2-2, when the proportion of the DNA substrate to the 5' sequencing adapter was 1 : 100, the concentration of the constructed PCR amplification pre-library was the lowest, at 0.0210 nM, and when the proportion was 1 : 4000, the concentration of the constructed PCR amplification pre-library was the highest, at 0.0836 nM.

Conclusion: When the proportion of the DNA substrate to the 5' sequencing adapter is from 1 : 100 to 1 : 4000, the methylation library for DNA which has been treated with hydrosulfite can be effectively constructed.

**Table 2-2**

| | | | | | |
|---|---|---|---|---|---|
| Proportion of DNA substrate: 5' sequencing adapter | 1 : 100 | 1 : 500 | 1 : 1000 | 1 : 2000 | 1 : 4000 |
| Concentration of library (nM) | 0.0210 | 0.0499 | 0.0641 | 0.0693 | 0.0836 |

### Example 3. The effect of different numbers of linear amplification cycles on methylation PCR amplification pre-library construction

(1) Adapter preparation: The tail-controlling adapter (001/002, as shown in Table 2) and the "5' sequencing adapter" with 7 overhanging Ns at the 3' terminus (012/015, as shown in Table 3) were prepared according to the adapter preparation method in Example 1.
(2) 20 ng of λ-DNA was treated with hydrosulfite by using a hydrosulfite treatment kit.
(3) The DNA product of step (2) was fragmented to 300 bp by using a focused ultrasonic apparatus (Covaris, Catalog No. S220), and retained for use.
(4) The DNA dephosphorylation reaction mixture was prepared as shown in Table 3-1. The reaction mixture was placed in a warm bath at 37°C for 30 minutes, treated at 95°C for 5 minutes, then immediately inserted in ice and incubated for 2 minutes, then retained for use.

**Table 3-1**

| DNA dephosphorylation reaction mixture | |
|---|---|
| DNA (from step (3)) | 17 µl |
| 10 x CutSmart buffer | 2 µl |
| FastAP thermo-sensitive alkaline phosphatase | 1 µl |
| Total volume | 20 µl |

(5) The tailing and ligation reaction mixture was prepared as shown in Table 3-2. The reaction mixtures were placed in a mixed bath at 37°C for 30 minutes, treated at 95°C for 5 minutes, and then kept at 4°C. The reaction mixtures were equally divided into 4 parts after the reaction was completed, each part being 10 µl, and retained use.

**Table 3-2**

| Tailing and ligation reaction mixture | |
|---|---|
| DNA (from step (4)) | 20 µl |
| Enzyme-free water | 4 µl |
| 2.5 x green buffer plus 12.5% PEG 8000 | 8 µl |
| 2 mM dATP plus 2 mM β-nicotinamide adenine dinucleotide | 2 µl |
| 10 µM tail-controlling adapter (001/002) | 2 µl |
| 1 : 33 diluted TdT enzyme (1 µl TdT enzyme plus 32 µl EB buffer) | 2 µl |
| 1 : 6 diluted *Escherichia coli* DNA ligase (1 µl TdT enzyme plus 5 µl EB buffer) | 2 µl |
| Total volume | 40 µl |

(6) The reaction mixture for linear amplification as shown in Table 3-3 was prepared, and ran according to the PCR amplification program as shown in Table 3-4, wherein the number of reaction cycles for 95°C for 30 seconds, 60°C for 30 seconds and 68°C for 1 minute were 4, 6, 8 and 12, respectively. All the linear amplification product was purified and recovered by using 166 µl of 1 : 6 diluted Beckman Ampure XP magnetic beads (1 volume of Beckman Ampure XP magnetic beads plus 5 volumes of SB buffer) and 280 µl of 1.8 : 1 diluted SB buffer (1.8 volume of SB buffer plus 1 volume of enzyme-free water), then eluted by adding 12.5 µl of EB buffer. For each of the four numbers of linear amplification cycles above, 12.5 µl of eluent was divided into 5 µl/part, with each part being charged into a 200 µl PCR tube, and 2 parts were separated and used for a next-step reaction.

**Table 3-3**

| Reaction mixture for linear amplification | |
|---|---|
| DNA (from step (5)) | 10 µl |
| Enzyme-free water | 59 µl |
| 5 x Phusion HF buffer | 20 µl |
| 100 µM linear amplification primer (003) | 2 µl |
| 2.5 mM dNTP | 8 µl |
| Phusion U Hot Start DNA polymerase | 1 µl |
| Total volume | 100 µl |

**Table 3-4**

| Temperature | Time | Number of cycles |
|---|---|---|
| 95°C | 3 minutes | 1 |
| 95°C | 30 seconds | 4 or 6 or 8 or 12 |
| 60°C | 30 seconds | |
| 68°C | 1 minute | |
| 68°C | 5 minutes | 1 |
| 4°C | Preservation | 1 |

(7) The DNA prepared in step (6) was reacted at 95°C for 5 minutes, and immediately placed on ice for 2 minutes for use.
(8) The four reaction mixtures for the ligation of the "5' sequencing adapter" were prepared as shown in Table 3-5. The reaction mixtures were respectively placed in a warm bath at 25°C for 15 minutes. The ligated pieces of DNA were respectively recovered by using 17 µl of Beckman Ampure XP magnetic beads, and then eluted by adding 26 µl of adding enzyme-free water to obtain the PCR amplification pre-library.

**Table 3-5**

| "5' sequencing adapter" ligation reaction mixture | | | | |
|---|---|---|---|---|
| Number of linear amplification cycles | 4 | 6 | 8 | 12 |
| DNA (from step (7)) | 5 µl | 5 µl | 5 µl | 5 µl |
| Enzyme-free water | 3.2 µl | 2.8 µl | 2.4 µl | 1.6 µl |
| 2 x T4 DNA quick ligation reaction buffer | 10 µl | 10 µl | 10 µl | 10 µl |
| 25 µM "5' sequencing adapter" with 7 overhanging Ns (012/015) | 0.8 µl | 1.2 µl | 1.6 µl | 2.4 µl |
| T4 DNA quick ligase | 1 µl | 1 µl | 1 µl | 1 µl |
| Total volume | 20 µl | 20 µl | 20 µl | 20 µl |

(9) The molar concentration of the PCR amplification pre-library was detected according to the method described in Example 1.

Experiment results: As shown in Table 3-6, the number of linear amplification cycles were 4, 6, 8, and 12. The molar concentrations of the PCR amplification pre-library were 0.0553 nM, 0.0947 nM, 0.131 nM and 0.199 nM, respectively. When the number of linear amplification cycles was 12, the concentration of the constructed library was the highest.

**Table 3-6**

| | | | | |
|---|---|---|---|---|
| Number of linear amplification cycles used | 4 | 6 | 8 | 12 |
| Concentration of library (nM) | 0.0553 | 0.0947 | 0.131 | 0.199 |

Conclusion: The tailed deoxypolynucleotide substrate was subjected to linear amplification, with there being 4-12 cycles of linear amplification, and then ligated to the "5' sequencing adapter" with 7 overhanging Ns, This can obtain an effective methylation library.

### Example 4. NGS detection of the effect of different numbers of linear amplification cycles on the construction of methylation sequencing library Materials:

2 x high-fidelity hot start PCR mixture (KAPA Biosystems, Catalog No. KK2602)

### Method:

(1) Adapter preparation: The tail-controlling adapter with a random molecular tag (006/029, as shown in Table 4) and the "5' sequencing adapter" with 7 overhanging Ns at the 3' terminus (012/015, as shown in Table 3) were prepared according to the method described in Example 1.
(2) Preparation of methylation sequencing library: The methylation sequencing library was constructed according to the method described in Example 3 until the "5' sequencing adapter" ligation reaction was completed; then, the ligated DNA was recovered by using 17 µl Beckman Ampure XP magnetic beads, and the ligated DNA was then eluted by adding 20 µl enzyme-free water; 2 µl of eluent was diluted 10 times in 18 µl of enzyme-free water to obtain a ten-fold diluent and 2 µl of the ten-fold diluent was then diluted 10 times in 18 µl of enzyme-free water to obtain a hundred-fold diluent; and the obtained diluent was diluted sequentially to obtain a ten-thousand-fold diluent. 5.34 µl of the ten-thousand-fold diluent was taken out and retained for use.
(3) The PCR amplification reaction mixture was prepared as shown in Table 4-1 and ran according to the PCR amplification program as shown in Table 4-2. The PCR product was recovered by using 80 µl of Beckman Ampure XP magnetic beads and then eluted by adding 50 µl enzyme-free water, and then, 50 µl of the eluted product was recovered by using 40 µl of Beckman Ampure XP magnetic beads, and the product was finally eluted by adding 25 µl of EB buffer to obtain the final sequencing library.

**Table 4-1**

| PCR amplification reaction mixture | |
|---|---|
| DNA (from step (2)) | 5.34 µl |
| Enzyme-free water | 40.66 µl |
| 50 µM P5 PCR tag primer (each library uses one of the primers 018, 019, 020, 021, 022, and 023, and the concentration of each primer is 50 µM) | 2 µl (added separately) |
| 25 µM P7 PCR tag primer (each library uses one of the primers 024 and 025, and the concentration of each primer is 25 µM) | 2 µl (added separately) |
| 2 x high-fidelity hot start PCR mixture | 50 µl |
| Total volume | 100 µl |

Table 4-2

| PCR amplification program | | |
|---|---|---|
| Temperature | Time | Number of cycles |
| 98°C | 45 seconds | 1 |
| 98°C | 15 seconds | 28 |
| 60°C | 30 seconds | |
| 72°C | 30 seconds | |
| 72°C | 5 minutes | 1 |
| 4°C | Preservation | 1 |

(4) The distribution of library fragments was detected by using Agilent 2100 bioanalyzer (Agilent Technologies, Catalog No. G2939BA) and Agilent high-sensitivity DNA kit (Agilent Technologies, Catalog No. 5067-4626); the molar concentration of the library was detected by using a library quantification kit (KAPA Biosystems, Catalog No. KK4808) and a DNA quantification standard and premixed primer kit (KAPA Biosystems, Catalog No. KK4808).
(5) An Illumina-NS500 sequencer was used to sequence, in an 75PE mode, the library obtained in step (3); the software Cutadapt (v1.12) was used to remove the adapter sequence; the software Bwa-Meth (v0.2.0) was used to perform genomic comparison of the methylation sequencing sequence; the software package Sambamba (v0.5.4) was used to mark repeated sequences; finally, the software package bedtods (v2.25.0) was used to compile statistics on the sequencing depth.

The experimental results are as shown in Table 4-3 below. When the numbers of linear amplification cycles are 4, 6, 8 and 12, the concentrations of the constructed methylation sequencing libraries are 32.22 nM, 54.90 nM, 134.24 nM and 139.95 nM, respectively. When the amount of sequenced data shown in high-throughput sequencing results is 4 Mb, the library construction efficiencies are 30.35%, 48.36%, 70.36%, and 92.63%, respectively, and the variation coefficients are 0.455, 0.275, 0.999 and 0.637, respectively.

Specifically, the library is constructed from 1000 template λ-DNA genomes, and finally, an average of 100 unique sequencing fragments is obtained at each λ-DNA genome site by means of sequencing, which is referred to as a library construction efficiency of 10%. Statistics are compiled on number of unique sequencing fragments obtained at each λ-DNA genome site, and the average value and standard deviation thereof are calculated; and the "variation coefficient" is obtained by dividing the standard deviation by the average value. The variation coefficient represents the uniformity of the method for constructing a library; the lower the variation coefficient, the better the uniformity. The library construction efficiency refers to the effectiveness of the method for constructing a library; the higher the library construction efficiency, the better the effectiveness.

Conclusion: The numbers of linear amplification cycles being 4, 6, 8 and 12 can effectively complete linear amplification, and can be used to construct the methylation sequencing library for DNA treated with hydrosulfite. When the number of linear amplification cycles is 12, the library construction efficiency is the highest, and reaches 92.63%.

**Table 4-3**

| Type of DNA | λ-DNA | λ-DNA | λ-DNA | λ-DNA |
|---|---|---|---|---|
| Amount of DNA added | 2 ng | 2 ng | 2 ng | 2 ng |
| Number of linear amplification cycles | 4 | 6 | 8 | 12 |
| Amount of DNA added during PCR amplification | 5.34 µl of the ten-thou sand- fold diluent of 20 µl of the ligated purification product described in step (2) | 5.34 µl of the ten-thou sand- fold diluent of 20 µl of the ligated purification product described in step (2) | 5.34 µl of the ten-thou sand- fold diluent of 20 µl of the ligated purification product described in step (2) | 5.34 µl of the ten-thou sand- fold diluent of 20 µl of the ligated purification product described in step (2) |
| Concentration of library (nM) | 32.22 | 54.90 | 134.24 | 139.95 |
| Sequencer | Illumina-NS500 | Illumina-NS500 | Illumina-NS500 | Illumina-NS500 |
| Sequencing mode | 75PE | 75PE | 75PE | 75PE |
| Amount of sequenced data | 4 Mb | 4 Mb | 4 Mb | 4 Mb |
| Library construction efficiency | 30.35% | 48.36% | 70.36% | 92.63% |
| Variation coefficient | 0.455 | 0.275 | 0.999 | 0.637 |

### Example 5

Comparison of the difference in efficiency between the method of the present invention, the traditional method and the Swift method in constructing a methylation sequencing library

### Materials:

10 x end repairing buffer (New England Biolabs, Catalog No. B6052S, 50 mM Tris-hydrochloric acid, 10 mM magnesium chloride, 10 mM dithiothreitol, 1 mM adenosine triphosphate, 0.4 mM dATP, 0.4 mM dCTP, 0.4 mM dGTP and 0.4 mM dTTP, pH 7.5)
T4 DNA polymerase (Enzymatics, Catalog No. P7080L, 3 U/µL)
T4 polynucleotide kinase (Enzymatics, Catalog No. Y9040L, 10 U/µL)
10 x dA tailing buffer (New England Biolabs, Catalog No. B6059S, 10 mM Tris-hydrochloric acid, 10 mM magnesium chloride, 50 mM sodium chloride, 1 mM dithiothreitol and 0.2 mM dATP, pH 7.9)
Klenow fragment (Exo-) (Enzymatics, Catalog No. P7010-LC-L, 10 U/µL)
(1) Construction of λ-DNA methylation sequencing library using the method of the present invention
(1-1) Adapter preparation: The tail-controlling adapter (001/002, as shown in Table 2) and the "5' sequencing adapter" with 7 overhanging Ns at the 3' terminus (012/015, as shown in Table 3) were prepared according to the adapter preparation method in Example 1.
(1-2) 20 ng of λ-DNA was treated with hydrosulfite by using a hydrosulfite treatment kit.
(1-3) The DNA product of step (1-2) was fragmented to 300 bp by using a focused ultrasonic apparatus (Covaris, Catalog No. S220); and divided equally into two parts (i.e., two parallels) and retained for use.
(1-4) The DNA dephosphorylation reaction mixture was prepared as shown in Table 5-1. The reaction mixture was placed in a warm bath at 37°C for 30 minutes, treated at 95°C for 5 minutes, then immediately inserted in ice and incubated for 2 minutes and retained for use.

**Table 5-1**

| 3DNA dephosphorylation reaction reaction | |
|---|---|
| DNA (from step (1-3)) | 17 µl |
| 10 x CutSmart buffer | 2 µl |
| FastAP thermo-sensitive alkaline phosphatase | 1 µl |
| Total volume | 20 µl |

(1-5) The tailing and ligation reaction mixture was prepared as shown in Table 5-2. The reaction mixture was placed in a mixed bath at 37°C for 30 minutes, treated at 95°C for 5 minutes, and then kept at 4°C and retained for use.

**Table 5-2**

| Tailing and ligation reaction mixtures | |
|---|---|
| DNA (from step (1-4)) | 20 µl |
| Enzyme-free water | 4 µl |
| 2.5 x green buffer plus 12.5% PEG 8000 | 8 µl |
| 2 mM dATP plus 2 mM β-nicotinamide adenine dinucleotide | 2 µl |
| 10 µM tail-controlling adapter (001/002) | 2 µl |
| 1 :33 diluted TdT enzyme (1 µl TdT enzyme plus 32 µl EB buffer) | 2 µl |
| 1 : 6 diluted *Escherichia coli* DNA ligase (1 µl TdT enzyme plus 5 µl EB buffer) | 2 µl |
| Total volume | 40 µl |

(1-6) The reaction mixture for linear amplification as shown in Table 5-3 was prepared, and ran according to the PCR amplification program as shown in Table 5-4. After the reaction was completed, the linear amplification product was purified and recovered by using 166 µl of 1 : 6 diluted Beckman Ampure XP magnetic beads (1 volume of Beckman Ampure XP magnetic beads plus 5 volumes of SB buffer) and 280 µl of 1.8 : 1 diluted SB buffer (1.8 volume of SB buffer plus 1 volume of enzyme-free water), then eluted using 6.6 µl of EB buffer.

**Table 5-3**

| Reaction mixture for linear amplification | |
|---|---|
| DNA (from step (1-5)) | 40 µl |
| Enzyme-free water | 29 µl |
| 5 x Phusion HF buffer | 20 µl |
| 100 µM linear amplification primer (003) | 2 µl |
| 2.5 mM dNTP | 8 µl |
| Phusion U Hot Start DNA polymerase | 1 µl |
| Total volume | 100 µl |

**Table 5-4**

| Temperature | Time | Number of cycles |
|---|---|---|
| 95°C | 3 minutes | 1 |
| 95°C | 30 seconds | 12 |
| 60°C | 30 seconds | |
| 68°C | 1 minute | |
| 68°C | 5 minutes | 1 |
| 4°C | Preservation | 1 |

(1-7) The DNA eluent in step (1-6) were reacted at 95°C for 5 minutes, and immediately placed on ice for 2 minutes and retained for use.
(1-8) The reaction mixture for the ligation of the "5' sequencing adapter" was prepared as shown in Table 5-5. The reaction mixture was placed in a warm bath at 25°C for 15 minutes. The ligated DNA was recovered by using 17 µl of Beckman Ampure XP magnetic beads, and then eluted by adding 100 µl of enzyme-free water. 2 µl of eluent was diluted 10 times in 18 µl of enzyme-free water to obtain a ten-fold diluent, then, 2 µl of the ten-fold diluent was diluted 10 times in 18 µl of enzyme-free water to obtain a hundred-fold diluent, and the obtained diluent was sequentially diluted to obtain a ten-thousand-fold diluent. 5.34 µl of the ten-thousand-fold diluent was taken out and retained for use.

**Table 5-5**

| | |
|---|---|
| DNA (from step (1-7)) | 6.6 µl |
| 2 x T4 DNA quick ligation reaction buffer | 10 µl |
| 25 µM "5' sequencing adapter" with 7 overhanging Ns (012/015) | 2.4 µl |
| T4 DNA quick ligase | 1 µl |
| Total volume | 20 µl |

(1-9) The PCR amplification reaction mixture was prepared as shown in Ttable 5-6 and ran according to the PCR amplification program as shown in Table 5-7. The PCR product was recovered by using 80 µl of Beckman Ampure XP magnetic beads and then eluted by adding 50 µl enzyme-free water, and then, 50 µl of the eluted product was recovered by using 40 µl of Beckman Ampure XP magnetic beads, and same was finally eluted by adding 25 µl of EB buffer to obtain the final sequencing library.

**Table 5-6**

| PCR amplification reaction mixture | |
|---|---|
| DNA (from step (1-8)) | 5.34 µl |
| Enzyme-free water | 40.66 µl |
| 50 µM P5 PCR tag primer (023) | 2 µl (added separately) |
| 25 µM P7 PCR tag primer (each library uses primer 024 or 025, and the | 2 µl (added separately) |
| concentration of each primer is 25 µM) | |
| 2 x high-fidelity hot start PCR mixture | 50 µl |
| Total volume | 100 µl |

**Table 5-7**

| PCR amplification program | | |
|---|---|---|
| Temperature | Time | Number of cycles |
| 98°C | 45 seconds | 1 |
| 98°C | 15 seconds | 28 |
| 60°C | 30 seconds | |
| 72°C | 30 seconds | |
| 72°C | 5 minutes | 1 |
| 4°C | Preservation | 1 |

(1-10) The method for detecting the library concentration was the same as in Example 4.
(1-11) The Illumina-Nova sequencer was used to sequence, in a 150 PE mode, the library obtained in step (1-9). The analysis method was the same as step (5) in Example 4.
(2) Construction of a λ-DNA methylation sequencing library using the traditional method (The schematic diagram of the library construction process is shown in figure 4)
(2-1) Adapter preparation: the "traditional methylation sequencing adapter" was prepared according to the adapter preparation method in Example 1 (016/017, as shown in Table 5).
(2-2) 20 ng λ-DNA was taken, and the DNA was fragmented to 300 bp by using a focused ultrasonic apparatus and was equally divided into two parts (i.e., two parallels) and retained for use.
(2-3) The end repairing reaction mixture was prepared as shown in Table 5-8 and reacted at 20°C for 30 minutes. Then, the repaired DNA was recovered by adding 45 µl of Beckman Ampure XP magnetic beads, and eluted using 26 µl of enzyme-free water.

**Table 5-8**

| End repairing reaction mixture | |
|---|---|
| DNA (from step (2-2)) | 24 µl |
| 10 x end repairing buffer | 3 µl |
| T4 DNA polymerase | 1.5 µl |
| T4 polynucleotide kinase | 1.5 µl |
| Total volume | 30 µl |

(2-4) The dA tailing reaction mixture was prepared as shown in Table 5-9 and reacted at 37°C for 30 minutes. Then, the DNA, the dA tailing thereof being complete, was recovered by adding 45 µl of Beckman Ampure XP magnetic beads, and eluted using 12 µl of enzyme-free water.

**Table 5-9**

| dA tailing reaction mixture | |
|---|---|
| DNA (from step (2-3)) | 26 µl |
| 10 x dA tailing reaction buffer | 3 µl |
| Klenow fragment (Exo-) | 1 µl |
| Total volume | 30 µl |

(2-5) The adapter ligation reaction mixture was prepared as shown in Table 5-10 and reacted at 25°C for 15 minutes. Then, the DNA, the ligation of which to the adapter is complete, was recovered by adding 21 µl of Beckman Ampure XP magnetic beads, and eluted using 20 µl of enzyme-free water.

**Table 5-10**

| Adapter ligation reaction mixture | |
|---|---|
| DNA (from step (2-4)) | 12 µl |
| 2 x T4 DNA quick ligation buffer | 15 µl |
| 1.5 µM "traditional methylation sequencing adapter" (016/017) | 2 µl |
| T4 DNA quick ligase | 1 µl |
| Total volume | 30 µl |

(2-6) The DNA (from step (2-5)) was treated with hydrosulfite using the hydrosulfite treatment kit and eluted using 100 µl of enzyme-free water; 2 µl of the eluent was diluted according to the dilution method described in step (1-8) to obtain a ten-thousand-fold diluent; 5.34 µl of the ten-thousand-fold diluent was taken out and retained for use.
(2-7) The PCR amplification reaction mixture was prepared as shown in Table 5-11 and ran according to the PCR amplification program as shown in Table 5-12. The PCR product was recovered by using 40 µl of Beckman Ampure XP magnetic beads and then eluted by adding 50 µl enzyme-free water, and then 50 µl of the eluted product was recovered by using 40 µl of Beckman Ampure XP magnetic beads, and same was finally eluted by adding 25 µl of EB buffer to obtain the final sequencing library.

**Table 5-11**

| PCR amplification reaction mixture | |
|---|---|
| DNA (from step (2-6)) | 5.34 µl |
| Enzyme-free water | 17.66 µl |
| 2 x high-fidelity hot start methylation PCR mixture | 25µl |
| 20 µM P5 PCR primer (026) | 1 µl |
| 20 µM P7 PCR tag primer (each library uses primer 027 or 028, and the concentration of each primer is 20 µM) | 1 µl |
| Total volume | 50 µl |

**Table 5-12**

| PCR amplification program | | |
|---|---|---|
| Temperature | Time | Number of cycles |
| 98°C | 45 seconds | 1 |
| 98°C | 15 seconds | 28 |
| 62°C | 30 seconds | |
| 72°C | 30 seconds | |
| 72°C | 2 minutes | 1 |
| 4°C | Preservation | 1 |

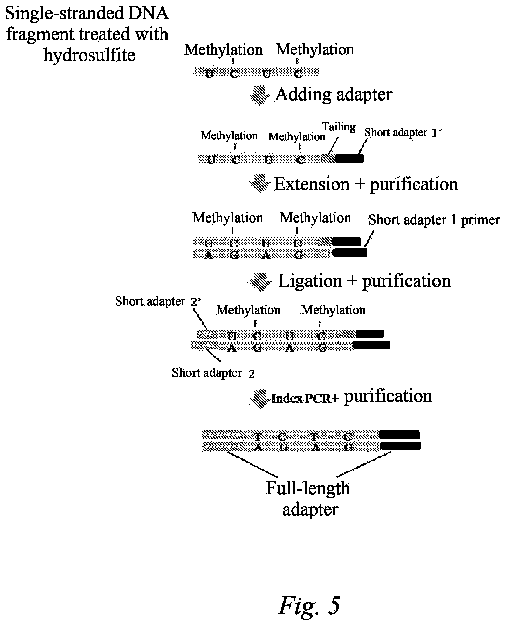

(2-8) The method for detecting the library concentration was the same as in Example 4.
(2-9) The Illumina-Nova sequencer was used to sequence, in a 150 PE mode, the library obtained in step (2-7). The analysis method was the same as step (5) in example 4.
(3) Construction of a λ-DNA methylation sequencing library using the Swift method (the schematic diagram of the library construction process is shown in figure 5)
(3-1) 20 ng of λ-DNA was treated with hydrosulfite by using a hydrosulfite treatment kit.
(3-2) The product of step (3-1) was fragmented to 300 bp by using a focused ultrasonic apparatus and divided equally into two parts (i.e., two parallels) and retained for use.
(3-3) The methylation library for DNA from step (3-2) was constructed according to the library construction steps of a Swift DNA methylation library construction kit (Swift Biosciences, Catalog No. 30024), with the difference being using 100 µl of enzyme-free water for elution after the 5' sequencing adapter was ligated and purified and during library construction (before Index PCR); 2 µl of the eluent was diluted according to the dilution method described in step (1-8) to obtain a ten-thousand-fold diluent; 5.34 µl of the ten-thousand-fold diluent was taken out and 14.66 µl of enzyme-free water was added to a final volume of 20 µl, and same was retained for use.
(3-4) The DNA from step (3-3) was subjected to PCR amplification according to the Index PCR steps in the Swift DNA methylation library construction kit (Swift Biosciences, Catalog No. 30024), and the Index used was 116 or 119 in the Index kit (Swift Biosciences, Catalog No. 36024), with the difference being that the number of PCR amplification cycles was 28.
(3-5) The PCR product from step (3-4) was purified and recovered according to the PCR product purification method shown in step (2-7) to obtain a final sequencing library.
(3-6) The molar concentration of the library was detected according to the method described in Example 4.
(3-7) The Illumina-Nova sequencer was used to sequence, in a 150PE mode, the library obtained in step (2-7). The analysis method was the same as step (5) in Example 4.

The experimental results are shown in Table 5-13. When respectively using the method of the present invention, the Swift method, and the traditional method, concentrations of the constructed methylation sequencing libraries are 18.684 nM, 1.641 nM and 0.146 nM, respectively. When the amount of sequenced data is 4 Mb, the library construction efficiencies are 56.1%, 22%, and 3.92%, respectively, and the variation coefficients are 0.463, 8.49, and 3.73, respectively.

**Table 5-13**

| Method for constructing a library | Method of the present invention | Swift Method | Traditional method |
|---|---|---|---|
| Type of DNA | λ-DNA | λ-DNA | λ-DNA |
| Initial amount of DNA added for constructing a library | 10 ng | 10 ng | 10 ng |
| Actual addition amount for PCR | 5.34 µl of the ten-thou sand- fold diluent of 100 µl of the ligated purification product described in step (1-8) | 5.34 µl of the ten-thou sand- fold diluent of 100 µl of the ligated purification product described in step (2-6) | 5.34 µl of the ten-thousand-fold diluent of 100 µl of the ligated purification product described in step (3-3) |
| Time point of hydrosulfite treatment | Before constructing library | Before constructing library | After constructing library |
| Number of linear amplification cycles | 12 | 1 | None |
| 5' sequencing adapter | with 7 overhanging random bases | with overhanging T | with overhanging T |
| Concentration of library | 18.684 nM | 1.641 nM | 0.146 nM |
| Sequencer | Illumina-nova | Illumina-nova | Illumina-nova |
| Sequencing mode | 150 PE | 150 PE | 150 PE |
| Amount of sequenced data | 4 Mb | 4 Mb | 4 Mb |
| Library construction efficiency | 56.1% | 22% | 3.92% |
| Variation coefficient | 0.463 | 8.49 | 3.73 |

Conclusion: The method of the present invention can be used to efficiently construct a methylation sequencing library of a small amount of genomic DNA, and the library construction efficiency and the uniformity of the method for constructing a library are far superior to those of Swift method and the traditional method.

## Claims

1. A method for constructing a library of a deoxypolynucleotide substrate, the method comprising the following steps:
(1) mixing a single strand of the deoxypolynucleotide substrate with the following substances to form a first mixture: a) a deoxynucleotide selected from one of dGTP, dCTP, dATP and dTTP; b) a terminal deoxynucleotidyl transferase and a DNA ligase; c) a tail-controlling component, wherein the tail-controlling component is a partially double-stranded nucleotide molecule composed of a polynucleotide homopolymer with a length of 5 to 20 nucleotides, and an X region, and a linker polynucleotide complementary to the X region, wherein the polynucleotide homopolymer is complementary to the deoxynucleotide in a);
(2) incubating the first mixture, wherein the 3' end of the single strand of the deoxypolynucleotide substrate undergoes a tailing reaction with the deoxynucleotides in a solution, and the 3' end of the substrate, to which the homopolymeric polynucleotide tail has been added, is ligated to the linker of the tail-controlling component to obtain a tailed substrate;
(3) adding a DNA polymerase, deoxynucleotides comprising dGTP, dCTP, dATP and dTTP, and a linear amplification primer to the reaction system of step (2) to form a second mixture;
(4) incubating the second mixture, wherein the tailed substrate obtained from step (2) is used as a template to carry out a first linear extension reaction, a complementary strand of the substrate is synthesized and then melted, after the linear amplification primer is complementary to the substrate, a subsequent linear extension reaction is performed again, and the number of instances of linear extension reaction is not less than 3;
(5) melting the product of step (4);
(6) adding a 5' sequencing adapter and a DNA ligase to the solution of step (5) to form a third mixture;
(7) incubating the third mixture, wherein the 5' sequencing adapter is connected to the complementary strand of the substrate to prepare and obtain a DNA library.

2. The method of claim 1, wherein the deoxypolynucleotide in a) is dATP.

3. The method of any one of the preceding claims, wherein the tail-controlling component in step (1) comprises a polynucleotide homopolymer with a length of 5 to 13 nucleotides.

4. The method of any one of the preceding claims, wherein the tail-controlling component in step (1) comprises a polynucleotide homopolymer with a length of 7 to 10 nucleotides.

5. The method of any one of the preceding claims, wherein step (2) is performed at a temperature of 20°C-50°C, preferably 25°C-45°C, more preferably 25°C-37°C.

6. The method of any one of the preceding claims, wherein the linear amplification primer in step (3) is complementary to 3' terminus of the tailed substrate.

7. The method of claim 6, wherein the linear amplification primer in step (3) is complementary to the linker of the tail-controlling component.

8. The method of any one of the preceding claims, wherein in the first linear extension reaction in step (4), the primer used is a tail-controlling molecule.

9. The method of to any one of claims 1-7, wherein in the first linear extension reaction in step (4), the primer used is the linear amplification primer added in step (3).

10. The method of claim 9, wherein in the first linear extension reaction in step (4), the sequence complementary to the substrate in the tail-controlling component is degraded or competes with the linear amplification primer added in step (3).

11. The method of any one of the preceding claims, wherein the number of instances of the linear extension reaction in step (4) is not less than 4, preferably 4-50, further preferably 4-20, and more preferably 4-12.

12. The method of any one of the preceding claims, wherein a single-stranded polynucleotide with 0-50 random bases overhangs at the 3' terminus of one strand of the 5' sequencing adapter in step (6) and (7).

13. The method of claim 12, wherein a single-stranded polynucleotide with 0-30 random bases overhangs at the 3' terminus of one strand of the 5' sequencing adapter in step (6) and (7).

14. The method of claim 13, wherein a single-stranded polynucleotide with 2-30 random bases, preferably 2-17, more preferably 4-15, and more preferably 7-10, overhangs at the 3' terminus of one strand of the 5' sequencing adapter.

15. The method of any one of the preceding claims, wherein the polynucleotide homopolymer and the linker of the tail-controlling component comprise a 3' blocking group.

16. The method of any one of the preceding claims, wherein the 5' sequencing adapter comprises a 3' blocking group.

17. The method of any one of the preceding claims, wherein the blocking group is selected from one or more of the following components: a ribonucleotide, a carbon 3 spacer, a phosphate, a dideoxynucleotide, an amino group and inverted deoxythymidine.

18. The method of any one of the preceding claims, the linker polynucleotide in the tail-controlling component comprising a 5'-end phosphate and the 3'-end blocking group.

19. The method of any one of the preceding claims, the strand complementary to the strand containing the random bases in the 5' sequencing adapter comprising a 5'-end phosphate.

20. The method of any one of the preceding claims, wherein the deoxypolynucleotide substrate is a dephosphorylated polynucleotide sequence.

21. The method of any one of the preceding claims, wherein after step (4), the double-stranded and single-stranded deoxypolynucleotide are separated from the second mixture, and after melting, the obtained single-stranded deoxypolynucleotide is used in the subsequent steps.

22. The method of any one of the preceding claims, wherein after step (7), PCR amplification is performed on the prepared DNA.

23. The method of claim 22, wherein after step (7), the prepared DNA is purified, and after purification, PCR amplification is performed.

24. A kit, comprising:
a first component, comprising a deoxynucleotide selected from one of dGTP, dCTP, dATP and dTTP, a terminal deoxynucleotidyl transferase, a DNA ligase and a tail-controlling component, wherein the tail-controlling component is a partially double-stranded nucleotide molecule composed of a polynucleotide homopolymer with a length of 5 to 20 nucleotides, and an X region, and a linker polynucleotide complementary to the X region, and the polynucleotide homopolymer is complementary to the deoxynucleotide selected from one of dGTP, dCTP, dATP and dGTP;
a second component, comprising a DNA polymerase, deoxynucleotides comprising dGTP, dCTP, dATP and dTTP, and a linear amplification primer;
and a third component, comprising a 5' sequencing adapter and a DNA ligase.

25. The kit of claim 24, wherein in the first component, the deoxynucleotide selected from one of dGTP, dCTP, dATP and dTTP, is dATP.

26. The kit of claim 24 or 25, wherein the tail-controlling component comprises a polynucleotide homopolymer with a length of 5 to 13, 7 to 10, more preferably 7 to 9 nucleotides.

27. The kit of any one of claims 24-26, wherein the linear amplification primer is complementary to the 3' end of the tail-controlling component, preferably complementary to the linker of the tail-controlling component.

28. The kit according to any one of claims 24-27, wherein a single-stranded polynucleotide with 0-50 random bases, preferably with 2-30, preferably 2-17, more preferably 4-15, more preferably 7-10 random bases, overhangs at the 3' terminus of one strand of the 5' sequencing adapter.

29. The use of the kit of any one of claims 24-28 for constructing a library of a deoxypolynucleotide substrate.
